# EUROPÄISCHE PATENTSCHRIFT

(11) **EP 1 566 175 B1**
(45) Veröffentlichungstag und Bekanntmachung des Hinweises auf die Patenterteilung: **25.10.2006**
(21) Anmeldenummer: 05001165.9
(22) Anmeldetag: 21.01.2005
(51) Int. Cl.: A61K 31/15, A61K 31/045, A61P 17/00, A61P 17/02

(54) **Topische Zubereitung, enthaltend mindestens ein Aryloxim und Bisabolol**
Topical preparation comprising at least one aryloxime and bisabolol
Préparation topique contenant au moins un aryloxime et du bisabolol

(30) Priorität: 18.02.2004 DE 102004007966
(43) Veröffentlichungstag der Anmeldung: 24.08.2005
(73) Patentinhaber: Merck Patent GmbH, 64293 Darmstadt (DE)
(72) Erfinder: Bünger, Joachim, Dr., 64853 Otzberg-Hering (DE); Axt, Alexandra, 64380 Rossdorf (DE); Horstmann, Stefan, Dr., 64646 Heppenheim (DE)

(56) Entgegenhaltungen:
- EP-A- 1 066 821
- WO-A-01/54653

## Beschreibung

Die vorliegende Erfindung betrifft eine topische Zusammensetzung, enthaltend mindestens ein Aryloxim und Bisabolol, eine Mischung geeignet zur Herstellung der topischen Zusammensetzung enthaltend mindestens ein Aryloxim und Bisabolol und ein Verfahren zur Herstellung dieser topischen Zusammensetzung.

Bei vielen Krankheiten sind Entzündungen als Symptome zu beobachten, die entweder ursächlich oder infolge krankhafter Veränderungen sekundär in Erscheinung treten. Sie können ebenfalls durch chemische oder physikalische Noxen von außen hervorgerufen werden. Eine Entzündung ist ein multifunktioneller Vorgang von unterschiedlichen morphologischen und funktionellen Faktoren. Diese Faktoren betreffen dabei sowohl Störungen im zellulären Bereich, bei der Blutzirkulation, entzündungsbedingte Trans- und Exsudation, Infiltration und Proliferation. Im Gefolge dieser Störungen können weitere Veränderungen auftreten, so dass u.a. Spongiose, Akanthose oder Parakeratose auftreten.

Bei Auslösung, dem Ablauf und der Steuerung vieler dieser Vorgänge sind Mediatorsysteme beteiligt. So sind die von sensibilisierten T-Lymphozyten abgegebenen Lymphokinine maßgeblich mit einer großen Zahl biologischer Wirkungen an der zellulären Immunantwort beteiligt (Schöpf, E., Korting, G.W. (Herausgeber) Dermatologie u. Praxis, Bd. 1, Thieme: Stuttgart, New York (1980)). Weiterhin sind in diesem Zusammenhang die Wirkung von Kininen, aktivierten Komplementfaktoren, lysosomalen Enzymen, zyklischen Nukleotiden und verschiedenen epidermalen Faktoren bekannt. Eine besondere Rolle spielen die Prostaglandine und Leukotriene. Als Beispiel einer Prostaglandinwirkung ist eine chemotaktische Wirkung auf Leukotriene bekannt, die zeitlich nach den Kininen die Gefäßpermeabilität vermindert. Dagegen wirken Leukotriene chemotaktisch auf Granulozyten und beeinflussen die Kontraktibilität und Permeabilität von Gefäßen.

Ein UV-B Erythem wird, abgesehen von der Histaminfreisetzung, durch die Arachidonsäurekaskade vermittelt, wobei eine gesteigerte Cyclooxygenase-vermittelte Prostaglandinsynthese, insbesondere von PGE₂ und PGF₂, vorliegt. Der Lipoxygenaseweg über 5-HPETE und LTA4 führt zu wesentlichen Elementen der Entzündung, wie zelluläre Infiltration des entzündeten Gewebes und Ödembildung (Übersicht bei: Gallin, J., Goldstein, I.M., Snyderman R. (Herausgeber), Inflamation. Basic principles and clinical correlates, New York, Raven Press (1988)).

Zur Behandlung von Entzündungen sind verschiedene Wirkstoffe bekannt. Die größte Bedeutung in der Behandlung der vorstehend genannten Mechanismen, die zu unterschiedlichen Hauterkrankungen führen, haben Corticosteroide. Schwache bis mittelstarke Corticosteroide, meist nicht fluorierte Derivate des Hydrocortison, werden vorwiegend zur Therapie entzündlicher, allergischer und pruriginöser Hauterkrankungen eingesetzt. Allerdings treten bei der Behandlung mit Corticosteroiden in Abhängigkeit von dem angewandten Wirkstoff, der Art und Dauer der Behandlung unerwünschte Nebenwirkungen auf, die unbedingt bei der Anwendung dieser Substanzen beachtet und berücksichtigt werden müssen (Übersicht: Symposium in Topical Corticosteroids. In: Drugs Bd. 36, 5 (1988)). Aus diesen Gründen werden vorzugsweise nichtsteroidale entzündungshemmende Wirkstoffe eingesetzt, wobei von den bisher bekannten Substanzen die therapeutische Effektivität allerdings sehr begrenzt ist und meist unter der von Hydrocortison liegt. Das betrifft Wirkstoffe, wie Salicylsäure, Acetylsalicylsäure, Bufexamac, Bendazac, Phenylbutazon, Oxyphenbutazon, Diflumidon, Indometacin und teilweise auch Antihistaminika (Gloor, M.: Pharmakologie dermatologischer Externa. Springer Verlag Berlin Heidelberg New York, (1982)).

In der EP-A-0149 242 werden als Wirkstoffe u.a. zur Behandlung von Hauterkrankungen 1-(2-Hydroxyaryl)-alkan-1-on-oxime vorgeschlagen. Die Wirkstoffe können dabei oral, perlingual, rektal, parenteral, intravenös oder percutan sowie als Aerosol angewendet werden. Als pharmazeutische Zubereitung werden u.a. Suspensionen und Emulsionen, Pasten, Salben, Gele, Cremes und Lotionen genannt. In einem Beispiel wurde 2-Hydroxy-5-methyl-laurophenon-oxim intraperitonal appliziert, und die Hemmung des Carrageeninödems der Rattenpfote wurde untersucht.

Neben den Eigenschaften des Wirkstoffes und des morphologischen und funktionellen Zustandes der zu behandelnden Hautareale sind es vor allem die Eigenschaften des benutzten Vehikels, von denen die therapeutische Effektivität eines topisch applizierten Arzneimittels abhängig ist. Insgesamt wird ein ausreichendes, optimales Konzentrations-Zeit-Profil des Wirkstoffs in der erkrankten Hautschicht angestrebt.

Die EP-B-389 773 offenbart ein Verfahren zur Herstellung einer galenischen Zubereitung mit optimaler Bioverfügbarkeit des Wirkstoffes 2-Hydroxy-5-methyl-laurophenon-oxim. Dabei werden Gallensäuren als Resorptionsmittel verwendet. Als Gallensäuren können beispielsweise Desoxy- oder Dehydrocholsäure oder Gemische dieser in Form ihrer Salze verwendet werden. Die Formulierung kann in Form von Lösungen, Suspensionen, Kapseln, Granulaten, Tabletten oder Dragees erfolgen.

In der DE-A-41 16 123 werden topische Zusammensetzungen auf der Basis von 2-Hydroxy-5-methyl-laurophenon-oxim offenbart. Als Anwendungsgebiete werden die pharmazeutische Industrie, die Human- und Veterinärmedizin sowie die Kosmetik genannt. Neben dem Wirkstoff enthalten die Formulierungen weiterhin normalerweise in der Haut vorkommende hydratisierende Substanzen und/oder Moisturizer und/oder Pentetrationspromotoren und andere Stoffe. Die Wirkstoffkonzentration beträgt üblicherweise 0,001 bis 5 %, und die vorstehend genannten zusätzlichen Substanzen liegen in einer Menge von 0,1 bis 40 % vor. Als bevorzugte hydratisierende Substanz und/oder Moisturizer wird Harnstoff eingesetzt. Bevorzugte Pentetrationspromotoren beinhalten Propylenglykol und Gallensäure. Die topische Formulierung liegt in Form von W/O-Emulsionen, Hydrogelen oder Mischgelen in Form von wasserfreien und/oder wasserhaltigen und lipohilen Salben, wasserhaltigen lipophilen Salben oder nichtionischen Cremes, Pasten, Schüttelmixturen, Lotionen und Emulsionen vor. Die Einarbeitung von Harnstoff konnte die Penetration des Wirkstoffes in die verschiedenen Schichten der menschlichen Haut sowie die Liberation des Wirkstoffs aus der topischen Formulierung erhöhen. Der Zusatz von Propylenglykol und Natriumdesoxycholat als Penetrationspromotoren konnte diesen Effekt weiter verbessern.

Ein großes Problem bei der topischen Anwendung der vorstehend genannten Verbindungen stellt deren geringe Löslichkeit in kosmetischen und dermatologischen Formulierungen und deren Neigung zum Auskristallisieren dar. Insbesondere ist der Wirkstoff unter Wärmezufuhr in Emulsionssystemen löslich, jedoch wird nach dem Abkühlen sowohl in O/W- als auch in W/O-Emulsionen während der Lagerung ein Auskristallisieren dieses Wirkstoffes beobachtet. Selbst wenn der Wirkstoff innerhalb der Emulsionssysteme gelöst bleibt, kann es trotzdem nach Auftragen der Formulierung zu Auskristallisierungen in den Hautschichten kommen.

Zahlreiche Untersuchungen wurden durchgeführt, um optimale Wirkstoffzusammensetzungen zu erhalten, bei denen es zu einer Verbesserung der Löslichkeit und einem Verhindern des Auskristallisierens kommt. Obwohl Aryloxime, insbesondere 2-Hydroxy-5-methyl-laurophenonoxim, in verschiedenen Lösungsmitteln, wie Isopropanol, Aceton, Chloroform, 2-Phenylethanol und Triton-X100, gut löslich sind, eignen sich diese Träger nicht oder nur bedingt für eine Verwendung für kosmetische und dermatologische Formulierungen. Deshalb müssen die Träger nichttoxisch, nichtkarzinogen und hautverträglich sein und sollten weiterhin duftneutral sein. Weiterhin sollten diese Träger, z.B. Lösungsmittel oder Emulgatoren, mit dem Hauptanteil der topischen Zusammensetzung, d.h. einer wäßrigen Phase oder einer Ölphase, kompatibel sein und keine getrennte Phase bilden oder zur Ausfällung des Wirkstoffes führen.

WO 01/89469 lehrt topische Formulierungen mit Aryloximen und Emulgatoren, die die Löslichkeit und Stabilität der Aryloxime in der Formulierung erhöhen. Als Emulgatoren eingesetzt werden Ester aus Milchsäure, dessen Dimere oder Trimere mit C₅- bis C₁₆-Säuren, bevorzugt zusammen mit weiteren (Co)-Emulgatoren. Offenbart werden Liposome, in denen die Aryloxime liposomal verkapselt vorliegen, sodass ein Auskristallisieren des Aryloxims verhindert wird. Die Herstellung von Liposomen ist aber technisch aufwändig und führt damit zu einer Verteuerung des Produktes. Auch lassen sich Liposomen nicht in alle Arten von Formulierungen einarbeiten, sodass die Freiheit zur Entwicklung verschiedener Formulierungen stark eingeschränkt ist. Weiterhin sind Emulgatoren, auch wenn diese weitestgehend physiologisch verträglich sind, für die Haut immer eine Belastung. Daher sind auch aus grundsätzlichen Erwägungen heraus topische Formulierungen wünschenswert, die möglichst keine Emulgatoren oder, falls nicht darauf verzichtet werden kann, diese in möglichst geringer Konzentration enthalten.

EP-A-1066821 offenbart in den Beispielen 2 und 4 kosmetische Öl-in-Wasser-Emulsionen enthaltend 4-Hydroxy-3-Hydroxyenzaldoxim und Bisabolol.

WO 01/54653 erwähnt Bisabolol und 2-Hydroxy-5-methyllaurophenonoxim als anti-inflammatorische Stoffe und dass diese in kosmetischen Zubereitungen enthalten sein können. Eine Kombination dieser Stoffe ist nicht offenbart.

Überraschenderweise wurde nun gefunden, dass Aryloxime in hoher Konzentration stabil in topische Formulierungen eingearbeitet werden kann, wenn diese mit Bisabolol kombiniert wird.

Diesem Effekt liegt zu Grunde, dass Aryloxim/e in Bisabolol löslich ist/sind, Bisabolol für Aryloxim/e also als Lösungsmittel wirkt. In Kombination mit Bisabolol kann/können das/die Aryloxime in hoher Konzentration in topische Zusammensetzungen, insbesondere in kosmetischen und/oder pharmazeutische Formulierungen, eingearbeitet werden, ohne dass hierzu Additive wie organische Lösungsmittel oder Emulgatoren zugesetzt werden müssen.

Vorteilhaft wirkt Bisabolol wie das Aryloxim entzündungshemmend und verstärkt bzw. ergänzt damit dessen Wirkung. Dies ermöglicht daher die Entwicklung von einfachen und damit kostengünstig herzustellenden Zusammensetzungen mit antientzündlicher Wirkung. Weiterhin ermöglicht die Verwendung der Kombination von Aryloxim und Bisabolol den Verzicht auf spezielle Emulgatoren, Alkohol, Tenside oder ähnlichem, sodass sich bei der Entwicklung der Formulierung mehr Freiheitsgrade ergeben und verträglichere Produkte entwickelt werden können.

Die erfindungsgemäße topische Zusammensetzung ist zur Prophylaxe und/oder Behandlung von Hauterkrankungen und/oder Entzündungsreaktionen der Haut geeignet. Weiterhin kann die erfindungsgemäße topische Zusammensetzung zur kosmetischen Pflege der Haut verwendet werden.

Auf Grund Anwesenheit von Bisabolol können topische Formulierungen zur Verfügung gestellt werden, in denen Aryloxim/e gleichmäßig verteilt und in hoher Konzentration enthalten ist/sind, sodass nach Applikation der Formulierung an die Haut das/die Aryloxim/e von dieser in hoher Menge aufgenommen werden können.

Gegenstand der Erfindung ist daher eine Zusammensetzung enthaltend mindestens ein Aryloxim der allgemeinen Formel (I) 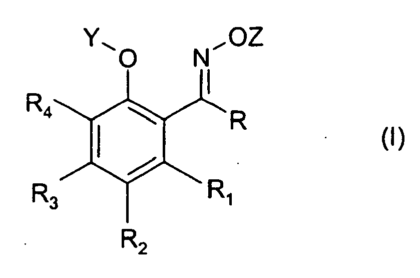 worin bedeuten:
- Y, Z: unabhängig voneinander H, C₁₋₁₈-Alkyl, C₂₋₁₈-Alkenyl, C₂₋₁₈-Carboxyalkyl, C₃₋₁₈-Carboxyalkenyl oder C₂₋₁₈-Alkanoyl;
- R: C₁₋₁₈-Alkyl, C₂₋₁₈-Alkenyl, C₃₋₈-Cycloalkyl, Aryl, Aralkyl, Heteroaryl, Heteroaralkyl oder kondensierte Systeme;
- R₁, R₂, R₃, R₄: unabhängig voneinander H, C₁₋₁₂-Alkyl, C₂₋₁₂-Alkenyl, C₁₋₁₂-Alkoxy, C₃₋₈-Cycloalkoxy, Aryl, Aryloxy, Aralkyl, Heteroaryl, Heteroaralkyl, Carboxy, Hydroxy, Chlor, Dialkylamin oder Sulfonyl,
und Bisabolol.

Alkyl, Alkenyl, Carboxyalkyl, Carboxyalkenyl, Alkanoyl, Cycloalkyl, Alkoxy, Aryl, Aryloxy und Aralkyl können unsubstituiert oder substituiert sein. Als Substituenten dieser Gruppen kommen vorzugsweise Alkyl, Alkoxy, Alkenyl, Aryl, Aryloxy, Aralkyl, Heteroaryl, Heteroaralkyl, Hydroxy, Carboxy, Carboxyalkyl, Dialkylamin, Sulfonyl und Kombinationen davon in Frage.

Alkyl bedeutet jeweils geradkettiges oder verzweigtes Alkyl und bedeutet daher bevorzugt Methyl, Ethyl, Propyl, Isopropyl, Butyl, Isobutyl, t-Butyl, Pentyl, Hexyl, Heptyl, Octyl, Nonyl, Decyl, Undecyl, Dodecyl, Tridecyl, Tetradecyl, Pentadecyl, Hexadecyl, Heptadecyl und Octadecyl.

Alkenyl bedeutet, dass in dem spezifizierten Alkylen eine oder mehrere Doppelbindungen vorhanden sein können.

Aryl steht für einen aromatischen C₆₋₂₀-Kohlenwasserstoffrest und bedeutet vorzugsweise Phenyl.

Aralkyl bedeutet eine mit Aryl substituierte Alkylgruppe und hat vorzugsweise die Bedeutung von Benzyl oder Phenethyl.

Cycloalkyl bedeutet eine cyclische Alkylgruppe und ist vorzugsweise Cyclopropyl, Cyclobutyl, Cyclopentyl, Cyclohexyl, Cycloheptyl oder Cyclooctyl.

Heteroaryl steht für einen aromatischen Ring mit Heteroatomen, vorzugsweise für einen stickstoffhaltigen Ring, wie Pyridinyl oder Pyrimidinyl.

Heteroaralkyl bedeutet eine mit Heteroaryl substituierte Alkylgruppe und ist vorzugsweise Pyridinylmethyl und Pyrimidinylmethyl.

Als kondensierte Systeme kommen vorzugsweise die Reste Naphthyl, Benzofuryl, Chinolinyl, Indolyl oder Cinnolinyl in Betracht.

Dialkylamin steht für NR₅R₆, wobei R₅ und R₆ gleich oder unterschiedlich sein können und C₁₋₁₂-Alkyl bedeuten.

Z und Y sind vorzugsweise unabhängig voneinander ein Wasserstoffatom, eine C₁₋₆-Alkylgruppe, die mindestens einen Substituenten, ausgewählt aus -OH, -COOH, -SO₃H oder NR₅R₆, besitzen kann, eine Alkanoylgruppe, dargestellt durch -C(O)R₇, worin R₇ eine C₁₋₆-Alkylgruppe, die mindestens einen Substituenten, ausgewählt aus -OH,
-COOH oder -SO₃H besitzen kann, oder eine CONHR₈-Gruppe, worin R₈ eine C₆₋₂₀-Arylgruppe bedeutet. Besonders bevorzugt sind Z und Y unabhängig voneinander ein Wasserstoffatom, -(CH₂)₁₋₆COOH,-CH₂CH(OH)CH₂OH, -(CH₂)₁₋₆SO₃H, -(CH₂)₁₋₆NR₅R₆ oder C(O)(CH₂)₁₋₆COOH.

Der Substituent R ist vorzugsweise eine C₁₋₁₂-Alkylgruppe, insbesondere bevorzugt sind C₁₋₅ und C₁₁-Alkylgruppen.

Der Substituent R₁ ist vorzugsweise ein Wasserstoff- oder Chloratom.

Der Substituent R₂ ist vorzugsweise ein Wasserstoff- oder Chloratom oder eine
C₁₋₆-Alkylgruppe. Besonders bevorzugt sind ein Wasserstoffatom, ein Chloratom und eine Methylgruppe.

Der Substituent R₃ ist vorzugsweise ein Wasserstoffatom oder eine C₁₋₆₋Alkylgruppe, eine C₁₋₆-Alkoxygruppe, eine O-Cyclohexylgruppe oder eine Benzylgruppe.

Der Substituent R₄ ist vorzugsweise ein Wasserstoff- oder Chloratom.

R₁, R₂, R₃ und R₄ können, wenn möglich, vorzugsweise mit -OH, -COOH, -SO₃H oder -NR₅R₆ substituiert sein, um z.B. die Wasserlöslichkeit zu erhöhen.

Bevorzugte Beispiele von Aryloximen der Formel (I), die in der erfindungsgemäßen Zusammensetzung enthalten sein können, umfassen: 4-Methyl-2-hydroxy-caprophenon-oxim, 5-Methyl-2-hydroxy-caprophenonoxim, 5-Methyl-2-hydroxy-caprophenon-(N-phenylcarbamoyl)-oxim, 5-Methyl-2-hydroxy-laurophenon-oxim (2-Hydroxy-5-methyl-laurophenonoxim), 3-Chlor-2-hydroxy-caprophenon-oxim, 4-Pentoxy-2-hydroxyacetophenon-oxim, 4-Decyloxy-2-hydroxy-acetophenon-oxim, 4-Benzyloxy-2-hydroxy-acetophenon-oxim, 4-Decyloxy-2-hydroxy-propiophenon-oxim, 4-Butoxy-5-n-hexyl-2-hydroxy-acetophenon-oxim, 4-Pentoxy-2-hydroxycaprophenon-oxim, 4-Decyloxy-2-hydroxy-caprophenon-oxim, 4-Octyloxy-2-hydroxy-laurophenon-oxim, 4-Cyclohexyl-oxy-2-hydroxy-propiophenonoxim, 5-Chlor-2-hydroxy-caprophenon-oxim, 3-Chlor-2-hydroxylaurophenon-oxim, 5-Chlor-2-hydroxy-laurophenon-oxim,4-Butoxy-2-hydroxy-acetophenon-oxim, 4-Dodecyloxy-2-hydroxy-propiophenon-oxim, 4-Hexadecyloxy-2-hydroxy-acetophenon-oxim, 4 Octadecyloxy-2-hydroxyacetophenon-oxim, 4-Decyloxy-2-hydroxy-laurophenon-oxim, sowie die folgenden Oximderivate von 2-Hydroxy-5-methyl-laurophenon-oxim:

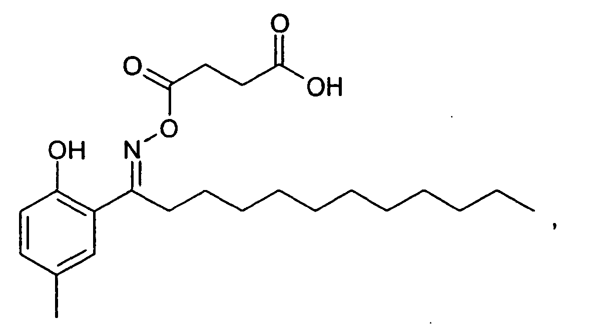

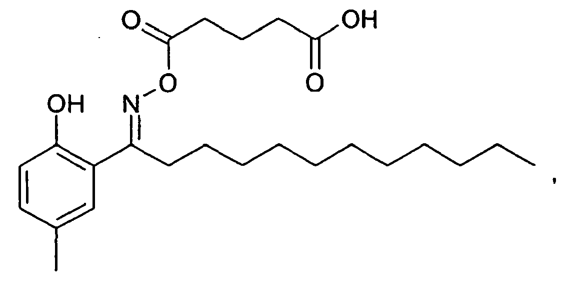

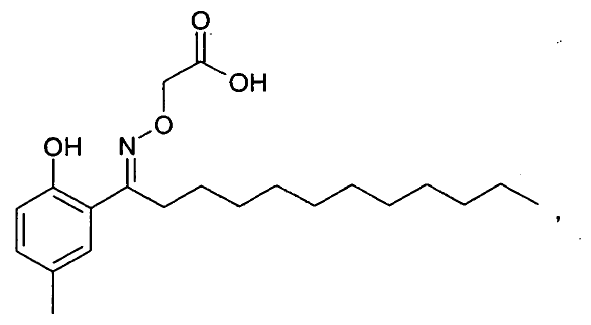

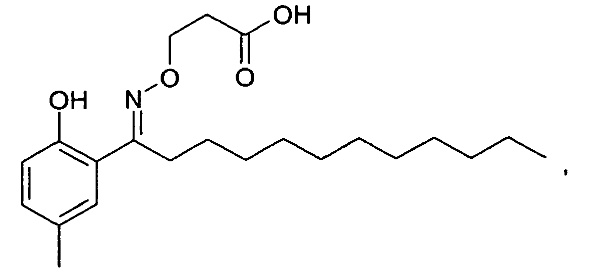

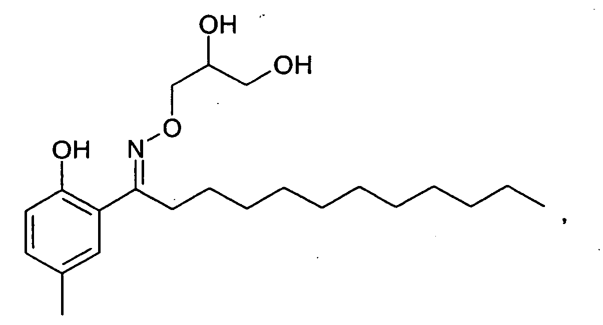

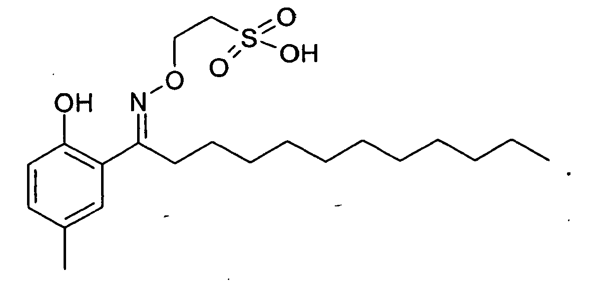

Besonders bevorzugt enthält die erfindungsgemäße topische Zusammensetzung 2-Hydroxy-5-methyl-laurophenon-oxim und/oder seine vorstehend genannten Oximderivate.

Das/die Aryloxim/e liegt/liegen in der erfindungsgemäßen topischen Zusammensetzung in einer ausreichenden Menge vor, um für eine kosmetische oder dermatologische Anwendung geeignet zu sein. Üblicherweise ist/sind Aryloxim/e in der erfindungsgemäßen topischen Zusammensetzung in einer Menge von insgesamt 0,001 bis 5 Gew.-%, vorzugsweise 0,02 bis 2 Gew.-%, noch bevorzugter 0,05 bis 1,5 Gew.-%, enthalten.

Bisabolol ist der Hauptwirkstoff der Kamille und ist unter dem Handelsnamen RonaCare^{®} Bisabolol (Fa. Merck) erhältlich. Die Substanz schützt trockene Haut und wirkt anti-inflammatorisch. Bisabolol ist in der erfindungsgemäßen topischen Zusammensetzung üblicherweise in einer Menge von insgesamt 0,05 bis 30 Gew.-% enthalten, bevorzugt sind 0,1 bis 2 Gew.-%, noch bevorzugter 0,2 bis 0,5 Gew.-%.

Je nach Anwendung enthält die erfindungsgemäße topische Zusammensetzung Hilfs- und/oder Trägerstoffe, wie Trägermittel, Konservierungsstoffe, Stabilisatoren, Lösungsmittel, Vitamine, Färbemittel, Geruchsverbesserer, Filmbildner, Verdickungsmittel und Feuchthaltemittel.

Als Anwendungsform der erfindungsgemäßen topischen Zusammensetzung seien z.B. genannt: Lösungen, Suspensionen, Emulsionen, Pasten, Salben, Gele, Cremes, Lotionen, tensidhaltige Reinigungspräparate und Öle.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silikone, Bentonite, Kieselsäure, Talkum und Zinkoxid, Xanthangummi, Glycerin, Carboxypolymethylen oder Gemische dieser Stoffe.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethylacetat, Benzylalkohol, Benzylbenzoat, Propylenglykol, 1,3-Butylglykol, Öle, insbesondere Baumwollsaatöl, Erdnußöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Die Emulsionen können in verschiedenen Formen vorliegen. So können sie z.B. eine Emulsion oder Mikroemulsion vom Typ Wasser-in-Öl (W/O), oder vom Typ Öl-in-Wasser (O/W), oder eine multiple Emulsion, beispielsweise vom Typ Wasser-in-Öl-in-Wasser (W/O/W), darstellen.

Die Zubereitung kann auch als emulgatorfreie, disperse Formulierung vorliegen. Sie kann beispielsweise eine Hydrodispersion oder eine Pikkering-Emulsion darstellen.

Die Zubereitung kann auch als PIT-Emulsion oder als Hydrogel vorliegen und auch Liposomen, die beispielsweise Wirkstoffe umschliessen, enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Pasten, Salben, Gele und Cremes können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure, Talkum und Zinkoxid oder Gemische dieser Stoffe.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestem, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Weitere Anwendungsformen der erfindungsgemäßen Zubereitung sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Alle Verbindungen oder Komponenten, die in der erfindungsgemäßen Zubereitung verwendet werden können, sind entweder bekannt und käuflich erwerbbar oder können nach bekannten Verfahren synthetisiert werden.

Die Formulierung kann Hilfsstoffe enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglycol oder Glycerin, und ein Verdickungsmittel wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die Formulierung kann auch zum Schutz der Haare gegen photochemische Schäden verwendet werden, um Veränderungen von Farbnuancen, ein Entfärben oder Schäden mechanischer Art zu verhindern. Geeignet ist hierbei eine Konfektionierung als Shampoo, Lotion, Gel oder Emulsion zum Ausspülen, wobei die jeweilige Formulierung vor oder nach dem Shamponieren, vor oder nach dem Färben oder Entfärben bzw. vor oder nach der Dauerwelle aufgetragen wird. Es kann auch eine Formulierung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Formulierung kann außer Aryloxim und Bisabolol verschiedene, in diesem Mitteltyp verwendete Hilfsstoffe enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäße Zubereitung kann mit Hilfe von Techniken hergestellt werden, die dem Fachmann wohl bekannt sind.

Zum Schutz der Haut und/oder natürlicher oder sensibilisierter Haare vor Sonnenstrahlen wird auf die Haut oder die Haare eine kosmetische Zubereitung, enthaltend Aryloxim/e und Bisabolol aufgetragen. Als sensibilisierte Haare werden dabei Haare verstanden, welche einer chemischen Behandlung, wie einer Dauerwellenbehandlung, einem Färbe- oder Entfärbeprozeß unterzogen worden sind.

Bevorzugt enthält die erfindungsgemäße topische Zusammensetzung weiterhin mindestens ein Antioxidantien und/UV-Filter.

Neben den bekannten Wirkungen der Antioxidantien und UV-Filtern, wie einem Schutz vor Zellschädigung durch Radikale bzw. einem Schutz vor UV-Strahlung und deren schädlicher Wirkung, können die Antioxidantien und/oder UV-Filter den enthaltenen Wirkstoff Aryloxim/e weiter stabilisieren. Dies wirkt sich z.B. in vorteilhafter Weise in einer Erhöhung der Lagerstabilität der erfindungsgemäßen topischen Zusammensetzung aus.

In den erfindungsgemäßen topischen Zusammensetzungen können die aus der Fachliteratur bekannten Antioxidantien enthalten sein, z.B. Flavonoide, Coumaranone, Aminosäuren (z.B. Glycin, Histidin, Tyrosin, Tryptophan) und deren Derivate, Imidazole, (z.B. Urocaninsäure) und deren Derivate, Peptide, wie D,L-Carnosin, D-Carnosin, L-Carnosin und deren Derivate (z.B. Anserin), Carotinoide, Carotine (z.B. β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Diaurylthiodipropionat, Distearylthiodipropionat, Thiodipropiosäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin), ferner (Metall-) Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate, Vitamin C und Derivate (z.B. Ascorbylpalmitat, Magnesium-Ascorbylphosphat, Ascorbylacetat) sowie Koniferylbenzoat des Benzoeharzes, Rutinsäure und deren Derivate, α-Glycosylrutin, Ferulasäure, Furfurylidenglucitol, Carnosin, Butylhydroxyltoluol (BHT), Butylhydroxyanisol, Nordohydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid).

Mischungen von Antioxidantien sind ebenfalls zur Verwendung in den erfindungsgemäßen topischen Zusammensetzungen geeignet. Bekannte und käufliche Mischungen sind beispielsweise Mischungen, enthaltend als aktive Inhaltsstoffe Lecithin, L-(+)-Ascorbylpalmitat und Zitronensäure (z.B. Oxynex^{®} AP), natürliche Tocopherole, L-(+)-Ascorbylpalmitat, L-(+)-Ascorbinsäure und Zitronensäure (z.B. Oxynex^{®} K LIQUID), Tocopherolextrakte aus natürlichen Quellen, L-(+)-Ascorbylpalmitat, L-(+)-Ascorbinsäure und Zitronensäure (z.B. Oxynex^{®} L LIQUID), DL-□-Tocopherol, L-(+)-Ascorbylpalmitat, Zitronensäure und Lecithin (z.B. Oxynex^{®} LM) oder Butylhydroxytoluol (BHT), L-(+)-Ascorbylpalmitat und Zitronensäure (z.B. Oxynex^{®} 2004).

In einer bevorzugten Ausführungsform der Erfindung enthält die erfindungsgemäße topische Zusammensetzung als Antioxidans Butylhydroxytoluol.

In einer weiteren bevorzugten Ausführungsform enthält die erfindungsgemäße topische Zusammensetzung als Antioxidans eine oder mehrere Verbindungen, ausgewählt aus Flavonoiden und/oder Coumaranonen.
Als Flavanoide werden die Glycoside von Flavanonen, Flavonen, 3-Hydroxyflavonen (= Flavanolen), Auronen, Isoflavonen und Rotenoiden aufgefaßt (Römpp Chemie Lexikon, Band 9, 1993). Im Rahmen der vorliegenden Erfindung werden hierunter jedoch auch die Aglykone, d.h. die zuckerfreien Bestandteile, und die Derivate der Flavonoide und der Aglykone verstanden. Im Rahmen der vorliegenden Erfindung werden unter Coumaranonen auch deren Derivate verstanden.

Bevorzugte Flavonoide leiten sich von Flavanonen, Flavonen, 3-Hydroxyflavonen, Auronen und Isoflavonen, insbesondere von Flavanonen, Flavonen, 3-Hydroxyflavonen und Auronen, ab.

In einer weiteren bevorzugten Ausführungsform, insbesondere, wenn die Wasserlöslichkeit der Flavonoide und Coumaranone gesteigert werden soll, ist an die Hydroxyguppen eine Sulfat- oder Phosphatgruppe gebunden. Geeignete Gegenionen sind beispielsweise die Ionen der Alkali- oder Erdalkalimetalle, wobei diese z.B. aus Natrium oder Kalium ausgewählt werden.

In einer weiteren bevorzugten Ausführungsform werden die Flavonoide ausgewählt aus folgenden Verbindungen: 4,6,3',4'-Tetrahydroxyauron, Quercetin, Rutin, Isoquercetin, Anthocyanidin (Cyanidin), Eriodictyol, Taxifolin, Luteolin, Trishydroxyethylquercetin (Troxequercetin), Trishydroxyethylrutin (Troxerutin), Trishydroxyethylisoquercetin (Troxeisoquercetin), Trishydroxyethylluteolin (Troxeluteolin) sowie deren Sulfate und Phosphate.

Unter den Flavonoiden sind insbesondere Rutin und Troxerutin bevorzugt. Besonders bevorzugt ist Troxerutin.

Unter den Coumaranonen ist 4,6,3',4'-Tetrahydroxybenzylcoumaranon-3 bevorzugt.

Die Antioxidationsmittel werden in der Regel in einer Menge von 0,001 bis 5 Gew.%, vorzugsweise 0,5 bis 5 Gew.% in die erfindungsgemäßen topischen Zusammensetzungen eingearbeitet.

Als geeignete organische UV-Filter kommen alle dem Fachmann bekannten UVA- als auch UVB-Filter in Frage. Für beide UV-Bereiche gibt es viele aus der Fachliteratur bekannte und bewährte Substanzen, z.B.

Benzylidenkampferderivate, wie
- 3-(4'-Methylbenzyliden)-dl-kampfer (z.B. Eusolex^{®} 6300),
- 3-Benzylidenkampfer (z.B. Mexoryl^{®} SD),
- Polymere von N-{(2 und 4)-[(2-oxoborn-3-yliden)-methyl]-benzyl} acrylamid (z.B Mexoryl^{®} SW),
- N,N,N-Trimethyl-4-(2-oxoborn-3-ylidenmethyl)-anilinium-methylsulfat (z.B. Mexoryl^{®} SK) oder
- α-(2-Oxoborn-3-yliden)-toluol-4-sulfonsäure (z.B. Mexoryl^{®} SL),

Benzoyl- oder Dibenzoylmethane, wie
- 1-(4-tert-Butylphenyl)-3-(4-methoxyphenyl)propan-1,3-dion (z.B. Eusolex^{®} 9020)
   oder
- 4-Isopropyldibenzoylmethan (z.B. Eusolex^{®} 8020),

Benzophenone, wie
- 2-Hydroxy-4-methoxybenzophenon (z.B. Eusolex^{®} 4360) oder
- 2-Hydroxy-4-methoxybenzophenon-5-sulfonsäure und ihr Natriumsalz (z.B. Uvinul^{®} MS-40),

Methoxyzimtsäureester; wie
- p-Methoxyzimtsäure-2-ethylhexylester (z.B. Eusolex^{®} 2292),
- p-Methoxyzimtsäureisopentylester, z.B. als Gemisch der Isomere (z.B. Neo Heliopan^{®} E 1000),

Salicylatderivate, wie
- 2-Ethylhexylsalicylat (z.B. Eusolex^{®} OS),
- 4-Isopropylbenzylsalicylat (z.B. Megasol^{®}) oder
- 3,3,5-Trimethylcyclohexylsalicylat (z.B. Eusolex^{®} HMS),

4-Aminobenzoesäure und Derivate davon, wie
- 4-Aminobenzoesäure,
- 4-(Dimethylamino)-benzoesäure-2-ethylhexylester (z.B. Eusolex^{®} 6007),
- ethoxylierte 4-Aminobenzoesäureethylester (z.B. Uvinul^{®} P25),
und weitere Substanzen, wie
- 2-Cyano-3,3-diphenylacrylsäure-2-ethylhexylester (z.B. Eusolex^{®} OCR),
- 2-Phenylbenzimidazol-5-sulfonsäure sowie ihre Kalium-, Natrium- und Triethanolaminsalze (z.B. Eusolex^{®} 232),
- 3,3'-(1,4-Phenylendimethylen)-bis-(7,7-dimethyl-2-oxobicyclo[2.2.1]hept-1-ylmethansulfonsäure sowie ihre Salze (z.B. Mexoryl^{®} SX) und
- 2,4,6-Trianilino-(p-carbo-2'-ethylhexyl-1'-oxi)-1,3,5-triazin (z.B. Uvinul^{®} T 150).

Diese organischen UV-Filter werden in der Regel in einer Menge von 0,5 bis
10 Gew.%, vorzugsweise 1 bis 8 Gew.%, in die erfindungsgemäßen topischen Zusammensetzungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)-disiloxanyl)-propylphenol (z.B. Silatrizole^{®}),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb^{®} HEB),
- α-(Trimethylsilyl)-ω[(trimethylsilyl)oxy]poly[oxy(dimethyl)] und ca. 6% methyl[2-[p-[2,2-bis(ethoxycarbonyl]vinyl]phenoxy]-1-methylenethyl] und ca. 1,5% methyl[3-[p-[2,2-bis(ethoxycarbonyl)vinyl)phenoxy)-propenyl) und 0,1 bis 0,4% (methylhydrogen]silylen]] (n≈60) (z.B. Parsol^{®} SLX,
- 2,2'-Methylen-bis-(6-(2H-benzotriazol-2-yl)-4-(1,1,33-tetramethylbutyl)phenol (z.B. Tinosorb^{®} M),
- 2,2'-(1,4-Phenylen)bis-1H-benzimidazol-4,6-disulfonsäure, Mononatriumsalz,
- 2,2'-(1,4-Phenylen)bis-1H-benzimidazol-5-sulfonsäure, Mononatriumsalz,
- 2,2'-(1,4-Phenylen)bis-1H-benzimidazol-4,6-disulfonsäure, Monokaliumsalz,
- 2,4-bis-{[4-(2-Ethyl-hexyloxy)-2-hydroxyl]-phenyl}-6-(4-methoxyphenyl)-1,3,5-triazin (z.B. Tinosorb^{®} S).

Diese organischen Filter werden in der Regel in einer Menge von 0,5 bis 20 Gew.%, vorzugsweise 1 bis 15 Gew.%, in die erfindungsgemäßen topischen Zusammensetzung eingearbeitet.

Als anorganische UV-Filter sind solche aus der Gruppe der Titandioxide, z.B. gecoatetes Titandioxid (z.B. Eusolex^{®} T-2000 oder Eusolex^{®} T-Aqua), Zinkoxide (z.B. Sachtotec^{®}), Eisenoxide oder auch Ceroxide denkbar. Diese anorganischen UV-Filter werden in der Regel in einer Menge von 0,5 bis 20 Gew.-%, vorzugsweise 2 bis 10 Gew.-%, in die erfindungsgemäßen topischen Zusammensetzungen eingearbeitet.

Bevorzugte UV-Filter sind Zinkoxid, Titandioxid, 3-(4'-Methylbenzyliden)-dlkampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze.

Besonders bevorzugte UV-Filter sind Zinkoxid und Titandioxid.

Unter den Titandioxid enthaltenden erfindungsgemäßen topischen Zusammensetzungen sind diejenigen bevorzugt, die neben Titandioxid zusätzlich einen oder mehrere weitere UV-Filter, ausgewählt aus 3-(4'-Methylbenzyliden)-dl-kampfer, 1-(4-tert-Butylphenyl)-3-(4-methoxyphenyl)propan-1,3-dion, 4-Isopropyldibenzoylmethan, 2-Hydroxy-4-methoxybenzophenon, Methoxyzimtsäureoctylester, 3,3,5-Trimethylcyclohexylsalicylat, 4-(Dimethylamino)benzoesäure-2-ethylhexylester, 2-Cyano-3,3-diphenylacrylsäure-2-ethylhexylester, 2-Phenylbenzimidazol-5-sulfonsäure sowie ihre Kalium-, Natrium- und Triethanolaminsalze, enthalten.

Unter diesen Zusammensetzungen sind diejenigen insbesondere bevorzugt, die neben Titandioxid zusätzlich die UV-Filter 2-Hydroxy-4-methoxybenzophenon und/oder Methoxyzimtsäureoctylester enthalten.

Nach einer vorteilhaften Ausführungsform der Erfindung enthält die erfindungsgemäße topische Zusammensetzung neben Aryloxim/en und Bisabolol eine oder mehrere weitere entzündungshemmend wirkende Substanz/en. Beispielhaft genannt seien hier Allantoin, Hespiridin, Azulen, Tilirosid, 5,7-Dihydroxy-chromon und Pflanzenextrakte wie z.B. Myrrheextrakt, Rataniawurzelextrakt, Salbeiextrakt, Kamilleextrakt oder Extrakte von Emblica officinalis.

Nach einer weiteren vorteilhaften Ausführungsform der Erfindung enthält die erfindungsgemäße topische Zusammensetzung einen oder mehrere kompatible Solute. Es handelt sich dabei um Substanzen, die an der Osmoregulation von Pflanzen oder Mikroorganismen beteiligt sind und aus diesen Organismen isoliert werden können. Unter den Oberbegriff kompatible Solute werden dabei auch die in der Deutschen Patentanmeldung DE-A-10133202 beschriebenen Osmolyte gefasst. Geeignete Osmolyte sind beispielsweise die Polyole, Methylamin-Verbindungen und Aminosäuren sowie jeweils deren Vorstufen. Als Osmolyte werden im Sinne der Deutschen Patentanmeldung DE-A-10133202 insbesondere Substanzen aus der Gruppe der Polyole, wie beispielsweise myo-Inositol, Mannitol oder Sorbitol und/oder einer oder mehrere der nachfolgend genannten osmolytisch wirksamen Stoffe verstanden:
Taurin, Cholin, Betain, Phosphorylcholin, Glycerophosphorylcholine, Glutamin, Glycin, α-Alanin, Glutamat, Aspartat, Prolin, und Taurin. Vorstufen dieser Stoffe sind beispielsweise Glucose, Glucose-Polymere, Phosphatidylcholin, Phosphatidylinositol, anorganische Phosphate, Proteine, Peptide und Polyaminsäuren. Vorstufen sind z. B. Verbindungen, die durch metabolische Schritte in Osmolyte umgewandelt werden.

Vorzugsweise werden erfindungsgemäß als kompatible Solute Substanzen gewählt aus der Gruppe bestehend aus Pyrimidincarbonsäuren (wie Ectoin und Hydroxyectoin), Prolin, Betain, Glutamin, cyclisches Diphosphoglycerat, N.-Acetylornithin, Trimethylamine-N-oxid Di-myo-inositol-phosphat (DIP), cyclisches 2,3-diphosphoglycerat (cDPG), 1,1-Diglycerin-Phosphat (DGP), β-Mannosylglycerat (Firoin), β-Mannosylglyceramid (Firoin-A) oder/und Di-mannosyl-di-inositolphosphat (DMIP) oder ein optisches Isomer, Derivat, z.B. eine Säure, ein Salz oder Ester dieser Verbindungen oder Kombinationen davon eingesetzt.

Dabei sind unter den Pyrimidincarbonsäuren insbesondere Ectoin ((S)-1,4,5,6-Tetrahydro-2-methyl-4-pyrimidincarbonsäure) und Hydroxyectoin ((S,S)-1,4,5,6-Tetrahydro-5-hydroxy-2-methyl-4-pyrimidincarbonsäure und deren Derivate zu nennen. Diese Verbindungen stabilisieren Enzyme und andere Biomoleküle in wässrigen Lösungen und organischen Lösungsmitteln. Weiter stabilisieren sie insbesondere Enzyme gegen denaturierende Bedingungen, wie Salze, extreme pH-Werte, Tenside, Harnstoff, Guanidiniumchlorid und andere Verbindungen.

Unter den kosmetischen Anwendungen ist insbesondere die Verwendung von Ectoin und Ectoin-Derivaten zur Pflege von gealterter, trockener oder gereizter Haut zu nennen. So wird in der europäischen Patentanmeldung EP-A-0 671 161 insbesondere beschrieben, dass Ectoin und Hydroxyectoin in kosmetischen Zubereitungen wie Pudern, Seifen, tensidhaltigen Reinigungsprodukten, Lippenstiften, Rouge, Make-Ups, Pflegecremes und Sonnenschutzpräparaten eingesetzt werden. Dabei wird vorzugsweise eine Pyrimidincarbonsäure gemäß der unten stehenden Formel VI eingesetzt, 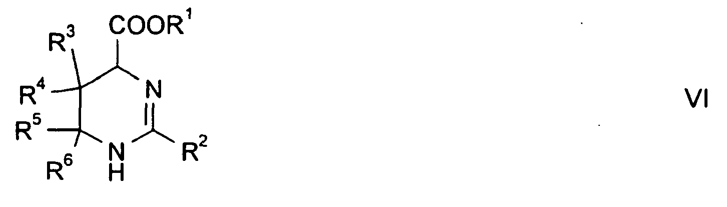 worin R¹ ein Rest H oder C1-8-Alkyl, R² ein Rest H oder C1-4-Alkyl und R³, R⁴, R⁵ sowie R⁶ jeweils unabhängig voneinander ein Rest aus der Gruppe H, OH, NH₂ und C1-4-Alkyl sind. Bevorzugt werden Pyrimidincarbonsäuren eingesetzt, bei denen R² eine Methyl- oder eine Ethylgruppe ist und R¹ bzw. R⁵ und R⁶ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%. Vorzugsweise werden die Pyrimidincarbonsäuren dabei in Verhältnissen von 100:1 bis 1:100 zu den Verbindungen der Formel I eingesetzt, wobei Verhältnisse im Bereich 1:10 bis 10:1 besonders bevorzugt sind.

Erfindungsgemäß insbesondere bevorzugt ist es dabei, wenn die kompatiblen Solute ausgewählt sind aus Di-myo-inositol-phosphat (DIP), cyclisches 2,3-diphosphoglycerat (cDPG), 1,1- Diglycerin-Phosphat (DGP), β-Mannosylglycerat (Firoin), β- Mannosylglyceramid (Firoin-A) oder/und Di-mannosyl-di-inositolphosphat (DMIP), Ectoin, Hydroxyectoin oder Mischungen davon.

Alle Verbindungen oder Komponenten, die in den Zubereitungen verwendet werden können, sind entweder bekannt und käuflich erwerbbar oder können nach bekannten Verfahren synthetisiert werden.

Enthält die erfindungsgemäße topische Zusammensetzung Aryloxim/e und Bisabolol in Kombination mit Antioxidantien und/oder UV-Filtern, Osmolyten und/oder weiteren Entzündungshemmer/n ergibt sich für die topische Zusammensetzung eine besonders stark entzündungshemmende und die Haut beruhigende Wirkung.

Bevorzugt werden das/die Aryloxim/e und Bisabolol vor dem Einarbeiten miteinander gemischt. Das Mischen wird vorzugsweise unter Rühren und bei einer erhöhten Temperatur durchgeführt. Die Temperatur während des Mischvorgangs beträgt vorzugsweise 20 bis 80°C, noch bevorzugter 40 bis 60°C. Besonders bevorzugt erfolgt die Herstellung der Mischung durch Auflösen des/der Aryloxims/Aryloxime in Bisabolol.

Die erhaltene Mischung ist lagerstabil. Die Mischung kann somit als Vorprodukt zur Herstellung der erfindungsgemäßen topischen Zusammensetzung eingesetzt und vertrieben werden und dann vom Hersteller der erfindungsgemäßen topischen Zusammensetzung als Mischung in einfacher Weise durch Mischen, beispielweise durch einfaches Einrühren, in die topische Zusammensetzung eingearbeitet werden. Gegenstand der Erfindung ist daher auch eine Mischung enthaltend mindestens ein Aryloxim der Formel (I) und Bisabolol, die zur Herstellung der topischen Zusammensetzung geeignet ist.

Bevorzugt enthält die erfindungsgemäße Mischung 1 bis 20 Gew.-% Aryloxim/e und 20 bis 99 Gew.-% Bisabolol.

Dabei kann es besonders vorteilhaft sein, wenn die Mischung 5 bis 15 Gew.-%, vorzugsweise 7 bis 12 Gew.-% und insbesondere bevorzugt ca. 10 Gew.-% Aryloxim/e und 80 bis 95 Gew.-%, vorzugsweise 88 bis 93 Gew.-% und insbesondere bevorzugt ca. 90 Gew.-% Bisabolol enthält.

In einer anderen Erfindungsvariante enthält die Mischung neben dem Bisabolol und dem/den Aryloxim/en noch weitere mit diesen Bestandteilen gut mischbare Öle. Dabei sind natürliche Öle, wie insbesondere Rizinus- oder Erdnussöl bevorzugt. Geeignet sind weiter Öle, wie Cetearyl Isononanoate, Butylphthalimide (and) Isopropylphthalimide, Isopropyl Isostearate, Ethoxydiglycol oder Ethyl Butylacetylaminopropionate (Bezeichnungen entsprechend der INCI-Nomenklatur). Die zusätzlichen Öle können dabei in Mengen von 0 bis 79 Gew.-% enthalten sein, wobei es erfindungsgemäß bevorzugt ist, wenn das zusätzliche Öl zu weniger als 50 Gew.-%, vorzugsweise zu maximal 30 Gew.-% an der Mischung beteiligt ist, mindestens jedoch in einer Menge von 1 Gew.-%, vorzugsweise von 5 Gew.-% vorliegt. In diesem Fall ist es bevorzugt, wenn die Mischung 5 bis 15 Gew.-%, vorzugsweise 7 bis 12 Gew.-% und insbesondere bevorzugt ca. 10 Gew.-% Aryloxim/e und 35 bis 94 Gew.-%, vorzugsweise 58 bis 88 Gew.-% und insbesondere bevorzugt ca. 80 Gew.-% Bisabolol enthält.

In der chemischen Verfahrenstechnik sind unter dem Begriff "Mischen" Grundoperationen zu verstehen, die der weitestgehenden Homogenisierung von Stoffen dienen. Es sollen Stoffströme so vereinigt werden, dass in Teilvolumina der entstehenden Mischung eine möglichst gleichmäßige Zusammensetzung der einzelnen Komponenten gegeben ist.

Eine Spezialform des Mischens stellt das Homogenisieren dar. Hierunter ist ein Vermischen von an sich nicht miteinander mischbaren Phasen zu verstehen. Unter Homogenisieren versteht man demnach ein Verändern des Verteilungszustandes und der Teilchengröße der inneren Phase von Emulsionen und Suspensionen, sodass mikroskopisch betrachtet ein homogenes System entsteht und sich die verteilte Phase ohne Einwirkung äußerer Kräfte nicht absetzt oder aufrahmt.

Unter Dispergieren ist ein Vermischen eines aus zwei oder mehreren Phasen bestehenden Stoffsystems zu verstehen, bei dem ein Stoff (disperse Phase) in einem anderen (Dispersionsmittel) in feinster Form verteilt (dispergiert) wird. Sowohl die Teilchen der dispersen Phase als auch das Dispersionsmittel können fest, flüssig oder gasförmig sein. Beispiele für Dispersionen sind Aerosole, Emulsionen, Suspensionen und Kolloide.

Eine andere in der Kosmetikherstellung übliche Art des Vermischens besteht im Emulgieren. Darunter ist ein Vermischen von zwei nicht oder nur wenig ineinander löslichen Flüssigkeiten zu verstehen, von denen die eine in der anderen fein verteilt wird. Die äußere Phase bezeichnet man als kontinuierliche Phase bzw. als Dispersionsmittel, die darin verteilte Flüssigkeit als die innere, diskontinuierliche oder disperse Phase. Kosmetische Emulsionen bestehen meistens aus einer wäßrigen polaren Phase und einer unpolaren Ölphase.

Unter Suspendieren wiederum ist das Verteilen sehr kleiner jedoch nicht molekularer Teilchen eines festen Stoffes oder einer Flüssigkeit zu verstehen. Suspensionen sind wie Emulsionen meist optisch trüb und neigen dazu, sich unter Einfluss der Schwerkraft abzusetzen.

Die vorstehend genannten Mischverfahren sind für die Herstellung der erfindungsgemäßen topischen Zusammensetzung geeignet. Besonders bevorzugt wird die erfindungsgemäße topische Zusammensetzung durch Homogenisieren, Dispergieren bzw. Emulgieren hergestellt.

Gegenstand der Erfindung ist auch ein Verfahren zur Herstellung der erfindungsgemäßen topischen Zusammensetzung, das dadurch gekennzeichnet ist, dass das/die Aryloxim/e und Bisabolol in diese als Mischung eingearbeitet werden.

Weiterer Gegenstand der Erfindung ist die Verwendung der erfindungsgemäßen mindestens ein Aryloxim und Bisabolol enthaltenden topischen Zusammensetzung zur Prophylaxe und/oder Behandlung von Hauterkrankungen und/oder Entzündungsreaktionen der Haut.

Weiterer Gegenstand der Erfindung ist die Verwendung der erfindungsgemäßen topischen zur kosmetischen Pflege der Haut.

Ebenfalls Gegenstand der Erfindung ist die Verwendung der Mischung enthaltend mindestens ein Arylocxim der Formel (I) und Bisabolol zur Herstellung der erfindungsgemäßen topischen Zusammensetzung.

Die erfindungsgemäße topische Zusammensetzung eignet sich zur Prophylaxe, Pflege und/oder Behandlung von Hauterkrankungen und/oder Entzündungsreaktionen der Haut. Insbesondere seien die folgenden Hauterkrankungen und Entzündungsreaktionen der Haut genannt:
- irritative Dermatitis,
- toxische Dermatitis,
- allergische Erkrankungen der Haut bzw. der Hautadnexen,
- entzündliche Erkrankungen der Haut bzw. der Hautadnexen,
- UV-Dermatitis und
- unterschiedliche Ekzemformen.

Die Ausführungsbeispiele, ohne hierauf beschränkt zu sein, erläutern die Erfindung.

### Beispiel 1

### Mischungen von Aryloxim und Bisabolol

Es werden Mischungen von 2-Hydroxy-5-methyl-laurophenon-oxim und Bisabolol hergestellt, indem die jeweilige Menge an 2-Hydroxy-5-methyllaurophenon-oxim unter Rühren in der jeweiligen Menge an Bisabolol gelöst wird.

| Inhaltsstoffe (Gew.-%) | | | | | |
|---|---|---|---|---|---|
| | | | | | |
| RonaCare^{®} LPO (2-Hydroxy-5-methyl-laurophenon-oxim) | 10,0 | 8,0 | 5,0 | 20,0 | 17,0 |
| RonaCare^{®} Bisabolol | 90,00 | 92,0 | 95,0 | 80,0 | 83,0 |

| Inhaltsstoffe (Gew.-%) | | | | | |
|---|---|---|---|---|---|
| | | | | | |
| RonaCare^{®} LPO (2-Hydroxy-5-methyl-laurophenon-oxim) | 10,0 | 12,0 | 15,0 | 10,0 | 5,0 |
| RonaCare^{®} Bisabolol | 45,0 | 50,0 | 66,0 | 80,0 | 70,0 |
| Rizinusöl | 45,0 | 35,0 | 29,0 | 10,0 | 25,0 |

Zur Herstellung der nachfolgenden topischen Zusammensetzungen werden jeweils Mischungen eingesetzt, in denen 2-Hydroxy-5-methyl-laurophenonoxim und Bisabolol im jeweils gewünschten Gewichtsverhältnis zueinander vorliegen.

### Beispiel 2

**Topische Zusammensetzung als O/W-Emulsion**

| | Rohstoff | Anbieter | INCI-Name | Gew.-% | Gew.-% |
|---|---|---|---|---|---|
| | | | | | |
| A | Emulgtor E 2155 | (1) | STEARETH-10, STEARETH-7, STEARYL ALCOHOL | 2.00 | 2.00 |
| | Teginacid H | (1) | GLYCERYL STEARATE, CETETH-20 | 2.00 | 2.00 |
| | Luvitol EHO | (2) | CETEARYL OCTANOATE | 10.00 | 10.00 |
| | Imwitor 900 | (3) | GLYCERYL STEARATE | 3.00 | 3.00 |
| | Cetiol | (4) | OLEYL OLEATE | 5.00 | 5.00 |
| | Lunacera M | (5) | MICROWAX | 1.00 | 1.00 |
| | Miglyol 812 N | (3) | CAPRYLIC/CAPRIC TRIGLYCERIDE | 3.00 | 3.00 |
| | Abil 100 | (1) | DIMETHICONE | 0.50 | 0.50 |
| | RonaCare^{®} Bisabolol | (6) | BISABOLOL | 0.30 | 0.30 |
| | RonaCare^{®} LPO | (6) | LAURYL P-CRESOL KETOXIME | 0.05 | 1.0 |
| | RonaCare^{®} Tocopherol acetat | (6) | TOCOPHERYL ACETATE | 0.50 | 0.50 |
| | Propyl-4-hydroxybenzoat | (6) | PROPYLPARABEN | 0.05 | 0.05 |
| | | | | | |
| B | dem. Wasser | | AQUA | ad 100 | ad 100 |
| | 1,2-Propandiol | (6) | PROPYLENE GLYCOL | 4.00 | 4.00 |
| | Methyl-4-hydroxybenzoat | (6) | METHYLPARABEN | 0.15 | 0.15 |

| | | | | | |
|---|---|---|---|---|---|
| (1) Degussa-Goldschmidt AG, (2) BASF AG, (3) Sasol Germany GmbH, (4) Cognis GmbH, (5) H.B. Fuller GmbH, (6) Merck KGaA/Rona^{®} | | | | | |

Die Phase A wird auf 75°C, Phase B auf 80°C erwärmt. B wird unter Rühren zu A gegeben. Anschließend wird das Gemisch homogenisiert (Ultra-Turrax) und abgekühlt.

### Beispiel 3

**Topische Zusammensetzung als sprühbare After Sun Lotion**

| | Rohstoff | Anbieter | INCI-Name | Gew. % |
|---|---|---|---|---|
| | | | | |
| A | dem. Wasser | | AQUA | ad 100 |
| | Glycerol (87%) | (1) | GLYCERIN | 4.00 |
| | LaraCare A-200 | (2) | GALACTOARABINA N | 0.25 |
| | | | | |
| B | Mirasil DM 100 | (3) | DIMETHICONE | 1.00 |
| | Cetiol OE | (4) | DICAPRYLYL ETHER | 3.00 |
| | RonaCare^{®} Bisabolol | (1) | BISABOLOL | 0.50 |
| | RonaCare^{®} LPO | (1) | LAURYL P-CRESOL KETOXIME | 0.05 |
| | | | | |
| C | Pemulen TR-2 | (5) | ACRYLATES/C10-30 ALKYL ACRYLATE CROSSPOLYMER | 0.15 |
| | | | | |
| D | dem. Wasser | | AQUA | 3.00 |
| | Tris(hydroxymethyl )-aminomethan | (1) | TROMETHAMINE | 0.15 |

| | | | | |
|---|---|---|---|---|
| (1) Merck KGaA/Rona^{®}, (2) Larex, (3) Rhodia GmbH, (4) Cognis GmbH, (5) Noveon | | | | |

Die Phasen A, B und D werden separat hergestellt, Phase C in Phase B dispergiert. Die Mischung aus B und C werden unter Rühren zu A gegeben, mit D neutralisiert und homogenisiert (Ultra-Turrax).

### Beispiel 4

**Topische Zusammensetzung als Hand- und Nagelcreme**

| | Rohstoff | Anbieter | INCl-Name | Gew.% |
|---|---|---|---|---|
| | | | | |
| A | flüssiges Paraffin | (1) | PARAFFINUM LIQUIDUM | 2.00 |
| | Arlamol HD | (2) | ISOHEXADECANE | 2.00 |
| | Isopropyl palmitate | (3) | ISOPROPYL PALMITATE | 3.00 |
| | Sojabohnenöl | (4) | GLYCINE SOJA (SOYBEAN OIL) | 0.50 |
| | Mirasil DM 350 | (5) | DIMETHICONE | 1.00 |
| | Lanette O | (3) | CETEARYL ALCOHOL | 1.00 |
| | Span 60 | (2) | SORBITAN STEARATE | 1.50 |
| | Montanov 68 | (6) | CETEARYL ALCOHOL, CETEARYL GLUCOSIDE | 4.00 |
| | RonaCare^{®} Bisabolol | (1) | BISABOLOL | 0.30 |
| | RonaCare^{®} LPO | (1) | LAURYL P-CRESOL KETOXIME | 0.015 |
| | | | | |
| B | dem. Wasser | | AQUA | ad 100 |
| | Glycerol (87%) | (1) | GLYCERIN | 10.00 |
| | Panthenol- D | (7) | PANTHENOL | 0.50 |
| | RonaCare^{®} Biotin | (1) | BIOTIN | 0.05 |
| | Konservierungsstoff | | | q.s. |
| | | | | |
| C | Rhodicare S | (5) | XANTHAN GUM | 0.30 |
| | | | | |
| D | Fragrance Bianca | (8) | PARFUM | 0.20 |

| | | | | |
|---|---|---|---|---|
| (1) Merck KGaA/Rona^{®}, (2) Uniqema, (3) Cognis GmbH, (4) Gustav Heess GmbH, (5) Rhodia GmbH, (6) Seppic, (7) Hoffmann-La Roche AG, (8) Symrise | | | | |

Phasen A und B werden jeweils auf 75°C erwärmt. Unter Aufrechterhaltung der Temperatur unter Rühren wird zunächst C wird zu B gegeben und nach Erhalt einer homogenen Mischung A zugegeben. Anschließend wird das Gemisch homogenisiert (Ultra-Turrax) und abgekühlt.

### Beispiel 5

**Topische Zusammensetzung als W/O-Emulsion**

| | Rohstoff | Anbieter | INCI-Name | Gew.% |
|---|---|---|---|---|
| | | | | |
| A | RonaCare^{®} LPO | (1) | LAURYL P-CRESOL ETOXIME | 0.05 |
| | RonaCare^{®} Bisabolol | (1) | BISABOLOL | 0.50 |
| | RonaCare^{®} LPO | (1) | LAURYL P-CRESOL KETOXIME | 0.05 |
| | Abil EM 90 | (2) | CETYL PEG/PPG-10/1 DIMETHICONE | 2.00 |
| | Bienenwachs weiß | (1) | CERA ALBA | 1.00 |
| | Cutina HR | (3) | HYDROGENATED CASTOR OIL | 0.50 |
| | flüss. Paraffin | (1) | PARAFFINUM LIQUIDUM (MINERAL OIL) | 9.50 |
| | Tegosoft DC | (2) | DECYL COCOATE | 8.00 |
| | Mirasil CM 5 | (4) | CYCLOMETHICONE | 6.00 |
| | | | | |
| B | Natriumchlorid | (1) | SODIUM CHLORIDE | 0.50 |
| | Glycerol (87%) | (1) | GLYCERIN | 3.00 |
| | Titriplex III | (1) | DISODIUM EDTA | 0.10 |
| | dem. Wasser | | AQUA | ad 100 |
| | Konservierungsstoffe | | | q.s. |
| | | | | |
| C | Emblica^{™} | (1) | PHYLLANTHUS EMBLICA FRUIT EXTRACT | 0.50 |
| | dem. Wasser | | AQUA | 10.00 |
| | | | | |
| D | Natriumhydroxid-Lsg. (10%) | (1) | SODIUM HYDROXIDE | 0.30 |
| | | | | |
| E | Ethanol 96% | (1) | ALCOHOL | 5.00 |
| | Fragrance | | PARFUM | q.s. |

| | | | | |
|---|---|---|---|---|
| (1) Merck KGaA/Rona®, (2) Degussa-Goldschmidt AG, (3) Cognis GmbH, (4) Rhodia GmbH | | | | |

Phasen A und B werden jeweils auf 80°C erwärmt. Unter Rühren wird B zu A gegeben und homogenisiert. Der pH-Wert von C wird mit D auf ca. 5,5 eingestellt. Anschließend wird das Gemisch der Phasen C/D und E dem Gemisch der Phasen A/B zugegeben, bei ca. 30°C homogenisiert (Ultra-Turrax) und bis zur Abkühlung auf Raumtemperatur gerührt.

### Beispiel 6

**Topische Zusammensetzung als emulgatorfreie Emulsion**

| | Rohstoff | Anbieter | INCl-Name | Gew.% | Gew.% |
|---|---|---|---|---|---|
| | | | | | |
| A | RonaCare® Bisabolol | (1) | BISABOLOL | 0.20 | 0.20 |
| | RonaCare^{®} LPO | (1) | LAURYL P-CRESOL KETOXIME | 0.04 | 0.10 |
| | Tegosoft DEC | (2) | DIETHYLHEXYL CARBONATE | 4.50 | 4.50 |
| | Prisorine 2039 | (3) | ISOSTEARYL ISOSTEARATE | 3.00 | 3.00 |
| | Ceraphyl368 | (4) | ETHYLHEXYL PALMITATE | 3.80 | 3.80 |
| | | | | | |
| B | Aristoflex AVC | (5) | AMMONIUM ACRYLOYLDIMETYL TAURATE/VP COPOLYMER | 1.00 | 1.00 |
| | | | | | |
| C | dem. Wasser | | AQUA | ad 100 | ad 100 |
| | 1,2-Propanediol | (1) | PROPYLENE GLYCOL | 5.00 | 5.00 |
| | Konservierungsstoff | | | q.s. | q.s. |
| | Harnstoff | (1) | UREA | 5.00 | 5.00 |
| | D-Panthenol | (6) | PANTHENOL | 0.20 | 0.20 |

| | | | | | |
|---|---|---|---|---|---|
| (1) Merck KGaA/Rona®, (2) Degussa-Goldschmidt AG, (3) Uniqema Chemie GmbH, (4) ISP Global Technologies, (5) Clariant GmbH, (6) Hoffmann-La Roche AG | | | | | |

Die Bestandteile der Phasen A und B werden jeweils getrennt voneinander gemischt, dann wird Phase B der Phase A zugegeben. Dem Gemisch der Phasen A und B wird unter Rühren Phase C zugegeben.

## Patentansprüche

1. Topische Zusammensetzung, enthaltend mindestens ein Aryloxim der Formel (I) 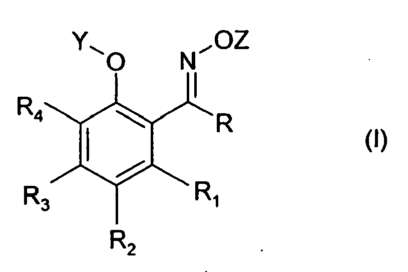 worin bedeuten:
Y, Z unabhängig voneinander H, C₁₋₁₈-Alkyl, C₂₋₁₈₋Alkenyl, C₂₋₁₈-Carboxyalkyl, C₃₋₁₈-Carboxyalkenyl oder C₂₋₁₈-Alkanoyl;
R C₁₋₁₈-Alkyl, C₂₋₁₈-Alkenyl, C₃₋₈-Cycloalkyl, Aryl, Aralkyl, Heteroaryl, Heteroaralkyl oder kondensierte Systeme;
R₁, R₂, R₃, R₄ unabhängig voneinander H, C₁₋₁₂-Alkyl, C₂₋₁₂₋Alkenyl, C₁₋₁₂-Alkoxy, C₃₋₈-Cycloalkoxy, Aryl, Aryloxy, Aralkyl, Heteroaryl, Heteroaralkyl, Carboxy, Hydroxy, Chlor, Dialkylamin oder Sulfonyl,
und Bisabolol.

2. Topische Zusammensetzung nach Anspruch 1, **dadurch gekennzeichnet, dass** mindestens ein Aryloxim der Formel (I) enthalten ist, das ausgewählt ist aus der Gruppe bestehend aus
4-Methyl-2-hydroxy-caprophenon-oxim, 5-Methyl-2-hydroxy-caprophenon-oxim, 5-Methyl-2-hydroxy-caprophenon-(N-phenylcarbamoyl)-oxim, 5-Methyl-2-hydroxy-laurophenon-oxim (2-Hydroxy-5-methyl-laurophenon-oxim), 3-Chlor-2-hydroxy-caprophenon-oxim, 4-Pentoxy-2-hydroxy-acetophenon-oxim, 4-Decyloxy-2-hydroxy-acetophenon-oxim, 4-Benzyloxy-2-hydroxy-acetophenon-oxim, 4-Decyloxy-2-hydroxy-propiophenon-oxim, 4-Butoxy-5-n-hexyl-2-hydroxy-acetophenon-oxim, 4-Pentoxy-2-hydroxy-caprophenon-oxim, 4-Decyloxy-2-hydroxy-caprophenon-oxim, 4-Octyloxy-2-hydroxy-laurophenon-oxim, 4-Cyclohexyl-oxy-2-hydroxy-propiophenon-oxim, 5-Chlor-2-hydroxy-caprophenon-oxim, 3-Chlor-2-hydroxy-laurophenon-oxim, 5-Chlor-2-hydroxy-laurophenon-oxim,4-Butoxy-2-hydroxy-acetophenon-oxim, 4-Dodecyloxy-2-hydroxy-propiophenon-oxim, 4-Hexadecyloxy-2-hydroxy-acetophenon-oxim, 4 Octadecyloxy-2-hydroxy-acetophenon-oxim, 4-Decyloxy-2-hydroxy-laurophenon-oxim, sowie den folgenden Oximderivaten von 2-Hydroxy-5-methyl-laurophenon-oxim: 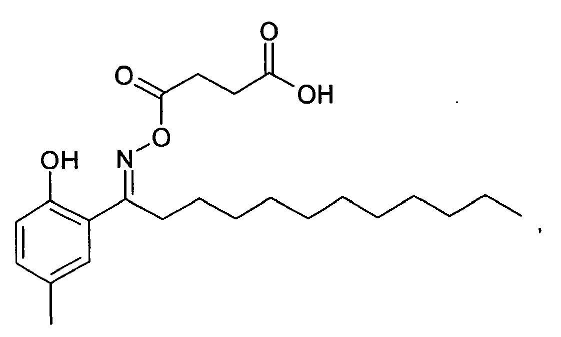 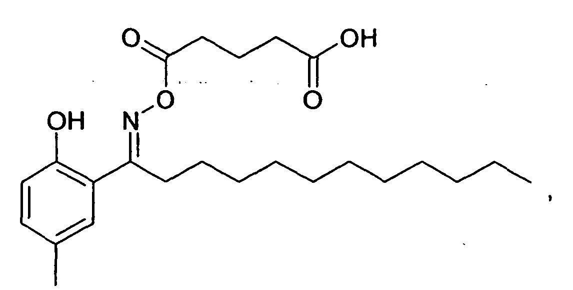 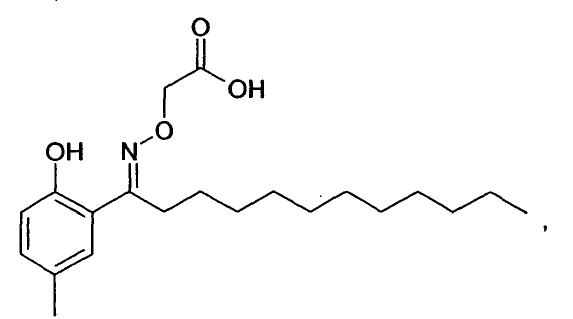 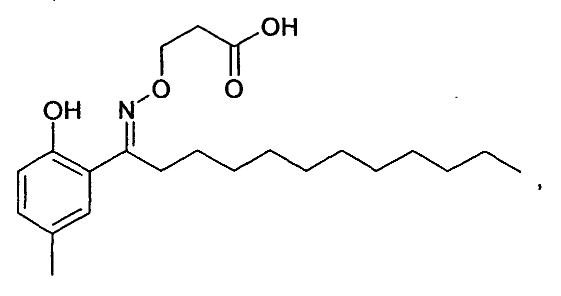 und

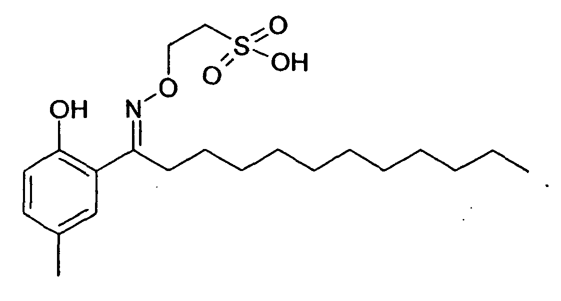

3. Topische Zusammensetzung nach Anspruch 2, **dadurch gekennzeichnet, dass** 2-Hydroxy-5-methyllaurophenonoxim und/oder mindestens eines der genannten Oximderivate enthalten ist.

4. Topische Zusammensetzung nach einem oder mehreren der Ansprüche 1 bis 3, **dadurch gekennzeichnet, dass** das/die Aryloxim/e in der Zusammensetzung in einer Menge von insgesamt 0,001 bis 5 Gew.-% enthalten ist/sind.

5. Topische Zusammensetzung nach einem oder mehreren der Ansprüche 1 bis 4, **dadurch gekennzeichnet, dass** diese Bisabolol in einer Menge von 0,05 bis 30 Gew.-% enthält.

6. Topische Zusammensetzung nach einem der Ansprüche 1 bis 5, **dadurch gekennzeichnet, dass** diese weiterhin mindestens ein Antioxidants und/oder UV-Filter, enthält.

7. Topische Zusammensetzung nach einem oder mehreren der Ansprüche 1 bis 6, **dadurch gekennzeichnet, dass** diese eine oder mehrere weitere entzündungshemmend wirkende Substanz/en enthält.

8. Topische Zusammensetzung nach einem oder mehreren der Ansprüche 1 bis 7, **dadurch gekennzeichnet, dass** diese einen oder mehrere kompatible Solute enthält.

9. Mischung enthaltend mindestens ein Aryloxim der Formel (I) nach Anspruch 1 und Bisabolol geeignet zur Herstellung der topischen Zusammensetzung gemäß einem oder mehreren der vorangehenden Ansprüche.

10. Mischung nach Anspruch 9, **dadurch gekennzeichnet, dass** diese 1 bis 20 Gew.-% Aryloxim/e und 80 bis 99 Gew.-% Bisabolol enthält.

11. Mischung nach Anspruch 10, **dadurch gekennzeichnet, dass** sie 5 bis 15 Gew.-%, vorzugsweise 7 bis 12 Gew.-% und insbesondere bevorzugt ca. 10 Gew.-% Aryloxim/e und 85 bis 95 Gew.-%, vorzugsweise 88 bis 93 Gew.-% und insbesondere bevorzugt ca. 90 Gew.-% Bisabolol enthält.

12. Mischung nach Anspruch 9, **dadurch gekennzeichnet, dass** diese 0 bis 79 Gew.-% zusätzliches Öl enthält, wobei es bevorzugt ist, wenn das zusätzliche Öl zu weniger als 50 Gew.-%, vorzugsweise zu maximal 30 Gew-% an der Mischung beteiligt ist, mindestens jedoch in einer Menge von 1 Gew.%, vorzugsweise von 5 Gew.-% vorliegt und die Mischung 5 bis 15 Gew.-%, vorzugsweise 7 bis 12 Gew.-% und insbesondere bevorzugt ca. 10 Gew.-% Aryloxim/e und 35 bis 94 Gew.-%, vorzugsweise 58 bis 88 Gew.-% und insbesondere bevorzugt ca. 80 Gew.-% Bisabolol enthält.

13. Verfahren zur Herstellung der topischen Zusammensetzung gemäß einem oder mehreren der Ansprüche 1 bis 8, **dadurch gekennzeichnet, dass** das/die Aryloxim/e und Bisabolol in diese als Mischung eingearbeitet werden.

14. Verwendung von Aryloxim der Formel (I) nach Anspruch 1 und/oder 2 in Kombination mit Bisabolol zur Herstellung einer topischen Zusammensetzung nach einem oder mehreren der Ansprüche 1 bis 8 zur Prophylaxe und/oder Behandlung von Hauterkrankungen und/oder Entzündungsreaktionen der Haut.

15. Nicht therapeutische Verwendung von Aryloxim der Formel (I) nach Anspruch 1 und/oder 2 in Kombination mit Bisabolol zur Herstellung einer topischen Zusammensetzung nach einem der Ansprüche 1 bis 8 zur kosmetischen Pflege der Haut.

16. Verwendung der Mischung nach einem oder mehreren der Ansprüche 9 bis 11 zur Herstellung der topischen Zusammensetzung nach einem oder mehreren der Ansprüche 1 bis 8.

## Claims

1. Topical composition comprising at least one aryl oxime of the formula (I) 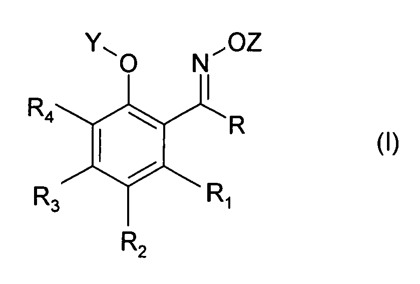 in which:
Y, Z, independently of one another, denote H, C₁₋₁₈-alkyl, C₂₋₁₈-alkenyl, C₂₋₁₈-carboxyalkyl, C₃₋₁₈-carboxyalkenyl or C₂₋₁₈-alkanoyl;
R denotes C₁₋₁₈-alkyl, C₂₋₁₈-alkenyl, C₃₋₈-cycloalkyl, aryl, aralkyl, heteroaryl, heteroaralkyl or fused systems;
R₁, R₂, R₃, R₄, independently of one another, denote H, C₁₋₁₂-alkyl, C₂₋₁₂-alkenyl, C₁₋₁₂-alkoxy, C₃₋₈-cycloalkoxy, aryl, aryloxy, aralkyl, heteroaryl, heteroaralkyl, carboxyl, hydroxyl, chlorine, dialkylamine or sulfonyl,
and bisabolol.

2. Topical composition according to Claim 1, **characterised in that** at least one aryl oxime of the formula (I) is present which is selected from the group consisting of
4-methyl-2-hydroxycaprophenone oxime, 5-methyl-2-hydroxycaprophenone oxime, 5-methyl-2-hydroxycaprophenone-(N-phenylcarbamoyl) oxime, 5-methyl-2-hydroxylaurophenone oxime (2-hydroxy-5-methyllaurophenone oxime), 3-chloro-2-hydroxycaprophenone oxime, 4-pentoxy-2-hydroxyacetophenone oxime, 4-decyloxy-2-hydroxyacetophenone oxime, 4-benzyloxy-2-hydroxyacetophenone oxime, 4-decyloxy-2-hydroxypropiophenone oxime, 4-butoxy-5-n-hexyl-2-hydroxyacetophenone oxime, 4-pentoxy-2-hydroxycaprophenone oxime, 4-decyloxy-2-hydroxycaprophenone oxime, 4-octyloxy-2-hydroxylaurophenone oxime, 4-cyclohexyloxy-2-hydroxypropiophenone oxime, 5-chloro-2-hydroxycaprophenone oxime, 3-chloro-2-hydroxylaurophenone oxime, 5-chloro-2-hydroxylaurophenone oxime, 4-butoxy-2-hydroxyacetophenone oxime, 4-dodecyloxy-2-hydroxypropiophenone oxime, 4-hexadecyloxy-2-hydroxyacetophenone oxime, 4-octadecyloxy-2-hydroxyacetophenone oxime, 4-decyloxy-2-hydroxylaurophenone oxime, and the following oxime derivatives of 2-hydroxy-5-methyllaurophenone oxime: 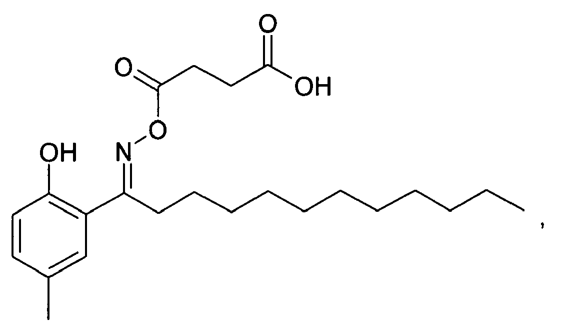 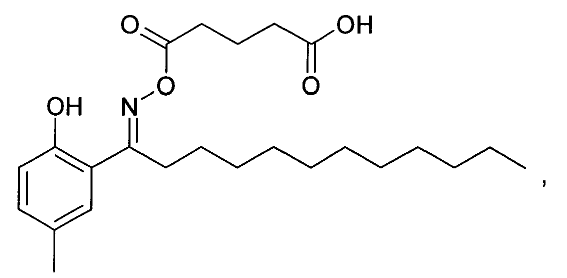 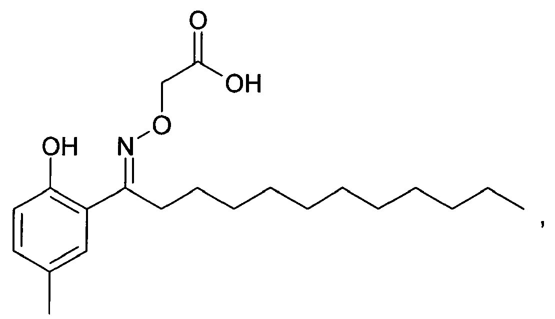 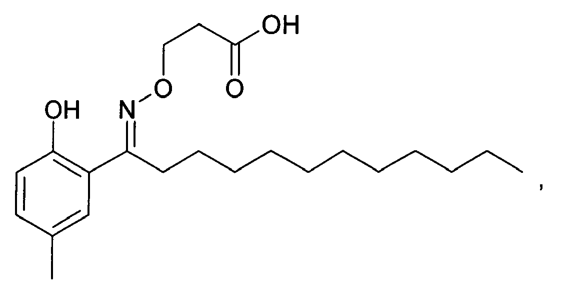 and

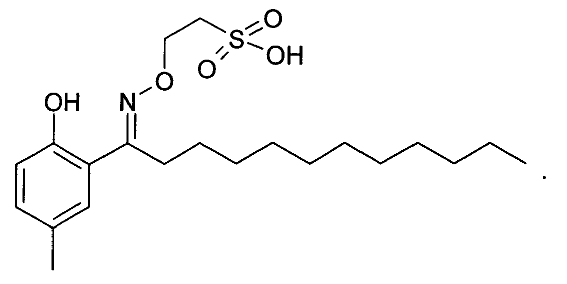

3. Topical composition according to Claim 2, **characterised in that** 2-hydroxy-5-methyllaurophenone oxime and/or at least one of the said oxime derivatives is present.

4. Topical composition according to one or more of Claims 1 to 3, **characterised in that** the aryl oxime(s) is (are) present in the composition in a total amount of 0.001 to 5% by weight.

5. Topical composition according to one or more of Claims 1 to 4, **characterised in that** it comprises bisabolol in an amount of 0.05 to 30% by weight.

6. Topical composition according to one of Claims 1 to 5, **characterised in that** it furthermore comprises at least one antioxidant and/or UV filter.

7. Topical composition according to one or more of Claims 1 to 6, **characterised in that** it comprises one or more further substance(s) having an inflammation-inhibiting action.

8. Topical composition according to one or more of Claims 1 to 7, **characterised in that** it comprises one or more compatible solutes.

9. Mixture comprising at least one aryl oxime of the formula (I) according to Claim 1 and bisabolol, which is suitable for the preparation of the topical composition according to one or more of the preceding claims.

10. Mixture according to Claim 9, **characterised in that** it comprises 1 to 20% by weight of aryl oxime(s) and 80 to 99% by weight of bisabolol.

11. Mixture according to Claim 10, **characterised in that** it comprises 5 to 15% by weight, preferably 7 to 12% by weight and particularly preferably about 10% by weight of aryl oxime(s) and 85 to 95% by weight, preferably 88 to 93% by weight and particularly preferably about 90% by weight of bisabolol.

12. Mixture according to Claim 9, **characterised in that** it comprises 0 to 79% by weight of additional oil, it being preferred for the additional oil to be present in the mixture to the extent of less than 50% by weight, preferably at most 30% by weight, but at least in an amount of 1% by weight, preferably 5% by weight, and the mixture comprises 5 to 15% by weight, preferably 7 to 12% by weight and particularly preferably about 10% by weight of aryl oxime(s) and 35 to 94% by weight, preferably 58 to 88% by weight and particularly preferably about 80% by weight of bisabolol.

13. Process for the preparation of the topical composition according to one or more of Claims 1 to 8, **characterised in that** the aryl oxime(s) and bisabolol are incorporated into the topical composition in the form of a mixture.

14. Use of aryl oxime of the formula (I) according to Claim 1 and/or 2 in combination with bisabolol for the preparation of a topical composition according to one or more of Claims 1 to 8 for the prophylaxis and/or treatment of skin diseases and/or inflammation reactions of the skin.

15. Non-therapeutic use of aryl oxime of the formula (I) according to Claim 1 and/or 2 in combination with bisabolol for the preparation of a topical composition according to one of Claims 1 to 8 for the cosmetic care of the skin.

16. Use of the mixture according to one or more of Claims 9 to 11 for the preparation of the topical composition according to one or more of Claims 1 to 8.

## Revendications

1. Composition topique comprenant au moins un oxime d'aryle de la formule (I) 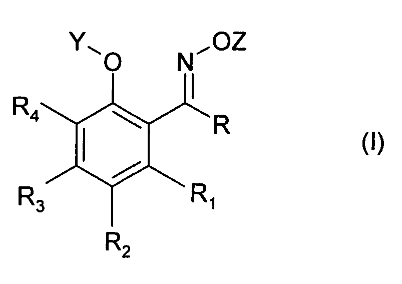 dans laquelle :
Y, Z, indépendamment l'un de l'autre, représentent H, C₁₋₁₈-alkyle, C₂₋₁₈-alkényle, C₂₋₁₈-carboxyalkyle, C₃₋₁₈-carboxyalkényle ou C₂₋₁₈-alkanoyle ;
R représente C₁₋₁₈-alkyle, C₂₋₁₈-alkényle, C₃₋₈-cycloalkyle, aryle, aralkyle, hétéroaryle, hétéroaralkyle ou des systèmes fusionnés ;
R₁, R₂, R₃, R₄, indépendamment les uns des autres, représentent H, C₁₋₁₂-alkyle, C₂₋₁₂-alkényle, C₁₋₁₂-alkoxy, C₃₋₈₋cycloalkoxy, aryle, aryloxy, aralkyle, hétéroaryle, hétéroaralkyle, carboxyle, hydroxyle, chlore, dialkylamine ou sulfonyle,
et du bisabolol.

2. Composition topique selon la revendication 1, **caractérisée en ce qu'**au moins un oxime d'aryle de la formule (I) est présent, lequel est choisi parmi le groupe comprenant
oxime de 4-méthyl-2-hydroxycaprophénone, oxime de 5-méthyl-2-hydroxycaprophénone, oxime de 5-méthyl-2-hydroxycaprophénone-(N-phénylcarbamoyl), oxime de 5-méthyl-2-hydroxylaurophénone (oxime de 2-hydroxy-5-méthyllaurophénone), oxime de 3-chloro-2-hydroxycaprophénone, oxime de 4-pentoxy-2-hydroxyacétophénone, oxime de 4-décyloxy-2-hydroxyacétophénone, oxime de 4-benzyloxy-2-hydroxyacétophénone, oxime de 4-décyloxy-2-hydroxypropiophénone, oxime de 4-butoxy-5-n-hexyl-2-hydroxyacétophénone, oxime de 4-pentoxy-2-hydroxycaprophénone, 4-décyloxy-2-hydroxycaprophénone, oxime de 4-octyloxy-2-hydroxylaurophénone, oxime de 4-cyclohexyloxy-2-hydroxypropiophénone, oxime de 5-chloro-2-hydroxycaprophénone, oxime de 3-chloro-2-hydroxylaurophénone, oxime de 5-chloro-2-hydroxylaurophénone, oxime de 4-butoxy-2-hydroxyacétophénone, oxime de 4-dodécyloxy-2-hydroxypropiophénone, oxime de 4-hexadécyloxy-2-hydroxyacétophénone, oxime de 4-octadécyloxy-2-hydroxyacétophénone, oxime de 4-décyloxy-2-hydroxylaurophénone, et les dérivés oxime qui suivent de oxime de 2-hydroxy-5-méthyllaurophénone : 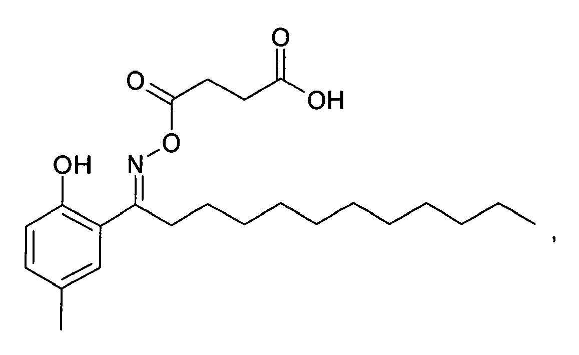 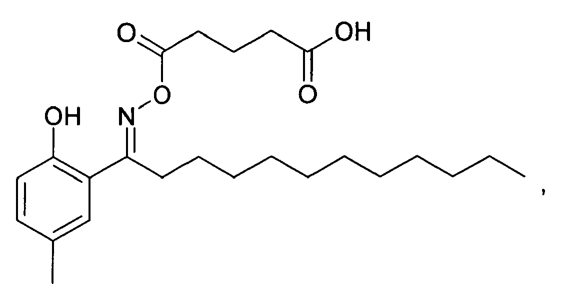 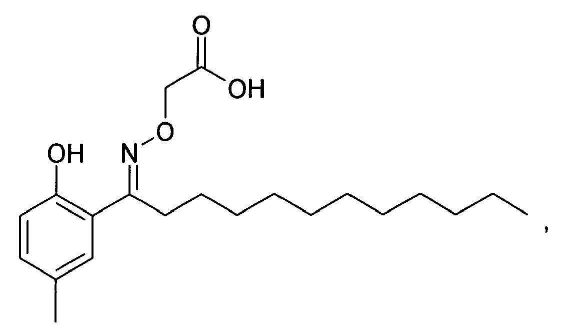 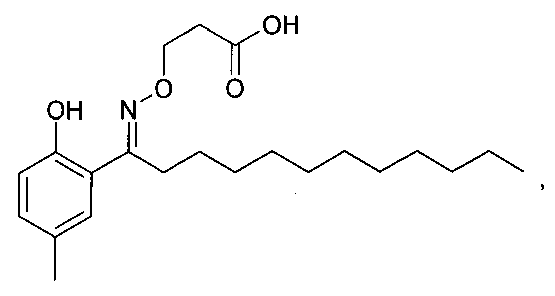 et

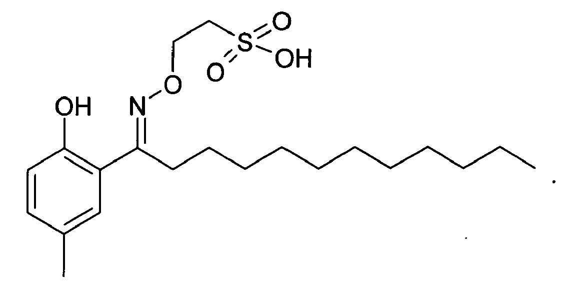

3. Composition topique selon la revendication 2, **caractérisée en ce** l'oxime de 2-hydroxy-5-méthyllaurophénone et/ou au moins l'un desdits dérivés oxime sont présents.

4. Composition topique selon une ou plusieurs des revendications 1 à 3, **caractérisée en ce que** l'/les oxime(s) d'aryle est (sont) présent(s) dans la composition selon une quantité totale de 0,001 à 5% en poids.

5. Composition topique selon une ou plusieurs des revendications 1 à 4, **caractérisée en ce qu'**elle comprend du bisabolol selon une quantité de 0,05 à 30% en poids.

6. Composition topique selon l'une des revendications 1 à 5, **caractérisée en ce qu'**elle comprend en outre au moins un antioxydant et/ou un filtre UV.

7. Composition topique selon une ou plusieurs des revendications 1 à 6, **caractérisée en ce qu'**elle comprend une ou plusieurs autres substance(s) présentant une action inhibitrice d'inflammation.

8. Composition topique selon une ou plusieurs des revendications 1 à 7, **caractérisée en ce qu'**elle comprend un ou plusieurs solutés compatibles.

9. Mélange comprenant au moins un oxime d'aryle de la formule (I) selon la revendication 1 et du bisabolol, lequel convient pour la préparation de la composition topique selon une ou plusieurs des revendications précédentes.

10. Mélange selon la revendication 9, **caractérisé en ce qu'**il comprend de 1 à 20% en poids d'oxime(s) d'aryle et de 80 à 99% en poids de bisabolol.

11. Mélange selon la revendication 10, **caractérisé en ce qu'**il comprend de 5 à 15% en poids, de préférence de 7 à 12% en poids et de façon particulièrement préférable, environ 10% en poids d'oxime(s) d'aryle et de 85 à 95% en poids, de préférence de 88 à 93% en poids et de façon particulièrement préférable, environ 90% en poids de bisabolol.

12. Mélange selon la revendication 9, **caractérisé en ce qu'**il comprend de 0 à 79% en poids d'huile additionnelle, étant entendu qu'il est préférable que l'huile additionnelle soit présente dans le mélange selon une quantité inférieure à 50% en poids, de préférence d'au plus 30% en poids, mais au moins selon une quantité de 1% en poids, de préférence de 5% en poids, et le mélange comprend de 5 à 15% en poids, de préférence de 7 à 12% en poids et de façon particulièrement préférable, environ 10% en poids d'oxime(s) d'aryle et de 35 à 94% en poids, de préférence de 58 à 88% en poids et de façon particulièrement préférable, environ 80% en poids de bisabolol.

13. Procédé pour la préparation de la composition topique selon une ou plusieurs des revendications 1 à 8, **caractérisé en ce que** l'/les oxime(s) d'aryle et le bisabolol sont incorporés dans la composition topique sous la forme d'un mélange.

14. Utilisation d'oxime d'aryle de la formule (I) selon la revendication 1 et/ou 2 en combinaison avec du bisabolol pour la préparation d'une composition topique selon une ou plusieurs des revendication 1 à 8 pour la prophylaxie et/ou le traitement de maladies de la peau et/ou de réactions inflammatoires de la peau.

15. Utilisation non thérapeutique d'oxime d'aryle de la formule (I) selon la revendication 1 et/ou 2 en combinaison avec du bisabolol pour la préparation d'une composition topique selon l'une des revendications 1 à 8 pour le soin cosmétique de la peau.

16. Utilisation du mélange selon une ou plusieurs des revendications 9 à 11 pour la préparation de la composition topique selon une ou plusieurs des revendications 1 à 8.
